# EUROPEAN PATENT SPECIFICATION

(11) **EP 4 180 127 B1**
(45) Date of publication and mention of the grant of the patent: **19.03.2025**
(21) Application number: 22206278.8
(22) Date of filing: 09.11.2022
(51) Int. Cl.: B01L 3/00, G01N 30/00

(54) **DETECTION CASSETTE AND DETECTION SYSTEM**
DETEKTIONSKASSETTE UND DETEKTIONSSYSTEM
CASSETTE DE DÉTECTION ET SYSTÈME DE DÉTECTION

(30) Priority: 11.11.2021 US 202163278111 P; 26.08.2022 TW 111132342
(43) Date of publication of application: 17.05.2023
(73) Proprietor: SKYLA Corporation, Hsinchu City 30044 (TW)
(72) Inventor: HE, Sz-Shian, 30044 Hsinchu City (TW)
(74) Representative: Becker, Eberhard

(56) References cited:
- EP-A1- 3 160 645
- EP-A1- 3 646 949
- EP-A1- 4 174 494
- EP-B1- 3 160 645
- WO-A1-2014/050946

## Description

### BACKGROUND

### Technical Field

The invention relates to a cassette, and particularly relates to a detection cassette and a detection system.

### Description of Related Art

In the field of biomedical testing, in order to detect different components in a specimen, such as plasma and blood cells in whole blood, it is usually necessary to perform tests on a plasma detection cassette and a blood cell detection cassette respectively. How to detect different components in the specimen by a single cassette is a research direction of the field. EP 3160645 A1 relates to a sample acquiring device for spectrophotometric measurement of high density lipoprotein (HDL) and total cholesterol (TC), triglycerides (TG) and glucose (FPG). EP 3646949 A1 relates to a detection cartridge including a detection tank, a sample tank, N containers, and first temporary tanks.

### SUMMARY

The invention is set out in the appended set of claims. The following disclosure serves a better understanding of the present invention. The invention is directed to a detection cassette, which is adapted to detect two components in a specimen (or referred to as a sample).

The invention provides a detection cassette adapted to detect a sample, the sample includes a first component and a second component. The detection cassette includes a sample injection hole, a first separation tank, a second separation tank, and a first detection tank and a second detection tank. The first separation tank is communicated with the sample injection hole, where a part of the sample is adapted to enter the first separation tank from the sample injection hole, and the part of the sample is separated into the first component and the second component in the first separation tank. The second separation tank is communicated with the sample injection hole, where another part of the sample is adapted to enter the second separation tank from the sample injection hole, and the another part of the sample is separated into the first component and the second component in the second separation tank. The first detection tank is communicated with the first separation tank, and the first component separated in the first separation tank flows to the first detection tank for detection. The second detection tank is communicated with the second separation tank, and the second component separated in the second separation tank flows to the second detection tank for detection. The detection cassette further comprises a first connection flow channel, connected between the first separation tank and the second separation tank.

In an embodiment of the invention, a density of the first component is less than a density of the second component, the first separation tank includes a first bottom portion and a first top portion, and the first component separated in the first separation tank is adapted to be located at the first top portion, and the second component separated in the first separation tank is adapted to be located at the first bottom portion. The second separation tank includes a second bottom portion and a second top portion, the first component separated in the second separation tank is adapted to be located at the second top portion, and the second component separated in the second separation tank is adapted to be located at the second bottom portion.

In an embodiment of the invention, the detection cassette further includes a first fluid tank and a first mixing tank. The first fluid tank is adapted to contain a first fluid. The first mixing tank is connected to the first top portion, and the first fluid tank is connected to the first top portion or the first mixing tank. The first mixing tank is located in a first direction of the first top portion and the first fluid tank, and the first component located at the first top portion and the first fluid located in the first fluid tank are adapted to be mixed in the first mixing tank.

In an embodiment of the invention, the detection cassette further includes a first quantitative tank and a first bending section. The first quantitative tank is connected to the first mixing tank. The first bending section includes a first section and a second section that are connected in a bending manner, where the first section is connected to the first quantitative tank, the second section is connected to the first detection tank, the first quantitative tank is located in a second direction of the first mixing tank and the first section, and the second direction is opposite to the first direction.

In an embodiment of the invention, the detection cassette further includes a quantitative flow channel and an overflow tank. The quantitative flow channel is connected between the sample injection hole and the first separation tank. The overflow tank is communicated with the second separation tank.

In an embodiment of the invention, the detection cassette further includes a first temporary storage tank, a second temporary storage tank and a second connection flow channel. The first temporary storage tank is communicated between the first separation tank and the first connection flow channel. The first temporary storage tank is located in a first direction of the second separation tank and in a second direction of the first separation tank, and the second direction is opposite to the first direction. The second temporary storage tank is communicated with the overflow tank. The second connection flow channel is communicated with the first temporary storage tank and the second temporary storage tank. The second connection flow channel and the second temporary storage tank are located in the second direction of the first temporary storage tank.

In an embodiment of the invention, there is a first necked section between the first bottom portion and the first top portion, and there is a second necked section between the second bottom portion and the second top portion.

In an embodiment of the invention, the detection cassette further includes a third temporary storage tank connected to the second top portion, the third temporary storage tank is located in a first direction of the second top portion, and the first component located at the second top portion is adapted to flow into the third temporary storage tank.

In an embodiment of the invention, the detection cassette further includes a second fluid tank and a second mixing tank. The second fluid tank is adapted to contain a second fluid and is communicated with the second bottom portion. The second mixing tank is communicated with the second bottom portion. The second bottom portion is located in a second direction of the second fluid tank, the second direction is opposite to the first direction, the second mixing tank is located in the second direction of the second bottom portion, and the second fluid in the second fluid tank is adapted to flow to the second bottom portion, so as to flow to the second mixing tank together with the second component located at the second bottom portion.

In an embodiment of the invention, the detection cassette further includes a third fluid tank and a third mixing tank. The third fluid tank is adapted to contain a third fluid. The third mixing tank is communicated with the second mixing tank and the third fluid tank, the third mixing tank is located in a third direction of the third fluid tank and the second mixing tank, and the third direction is orthogonal to the first direction and the second direction, and the third fluid in the third fluid tank and the second component and the second fluid in the second mixing tank are adapted to flow to the third mixing tank.

In an embodiment of the invention, the detection cassette further includes a second quantitative tank and a second bending section. The second quantitative tank is communicated with the third mixing tank. The second bending section includes a third section and a fourth section connected in a bending manner, where the third section is connected to the second quantitative tank, the fourth section is connected to the second detection tank, the second quantitative tank is located in a fourth direction of the third mixing tank and the third section, and the fourth direction is opposite to the third direction.

The invention further provides a detection system, including: a centrifugal platform having a carrier turntable driven by a driving module; and a detection cassette placed on the carrier turntable.

In an embodiment of the invention, the centrifugal platform further includes a sub turntable arranged on the carrier turntable and driven by another driving module, and the detection cassette is placed on the sub turntable.

Based on the above description, in the detection cassette of the invention, a part of the sample is adapted to enter the first separation tank from the sample injection hole of the detection cassette, and separated into the first component and the second component in the first separation tank. Another part of the sample is adapted to enter the second separation tank from the sample injection hole, and separated into the first component and the second component in the second separation tank. The first detection tank is communicated with the first separation tank, and the first component separated in the first separation tank flows to the first detection tank for detection. The second detection tank is communicated with the second separation tank, and the second component separated in the second separation tank flows to the second detection tank for detection. Therefore, the invention allows detections of the first component and the second component of one sample be conducted on one detection cassette, which effectively reduces detection cost and time, and increases utilization of the sample.

### BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.
FIG. 1 is a schematic diagram of a detection cassette according to an embodiment of the invention.
FIG. 2 is an exploded schematic view of the detection cassette of FIG. 1.
FIG. 3 is a schematic diagram of FIG. 2 from another viewing angle.
FIG. 4 to FIG. 14 are schematic diagrams of a flow process of a sample in the detection cassette of FIG. 1.

### DESCRIPTION OF THE EMBODIMENTS

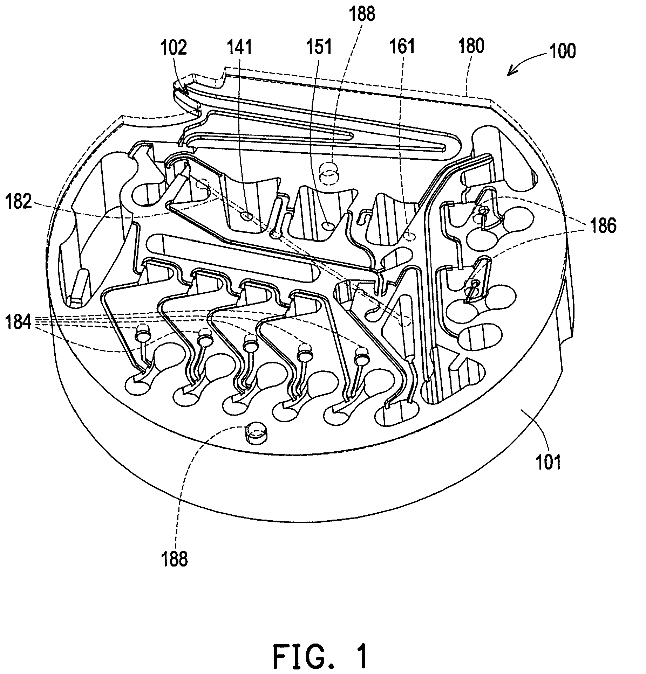

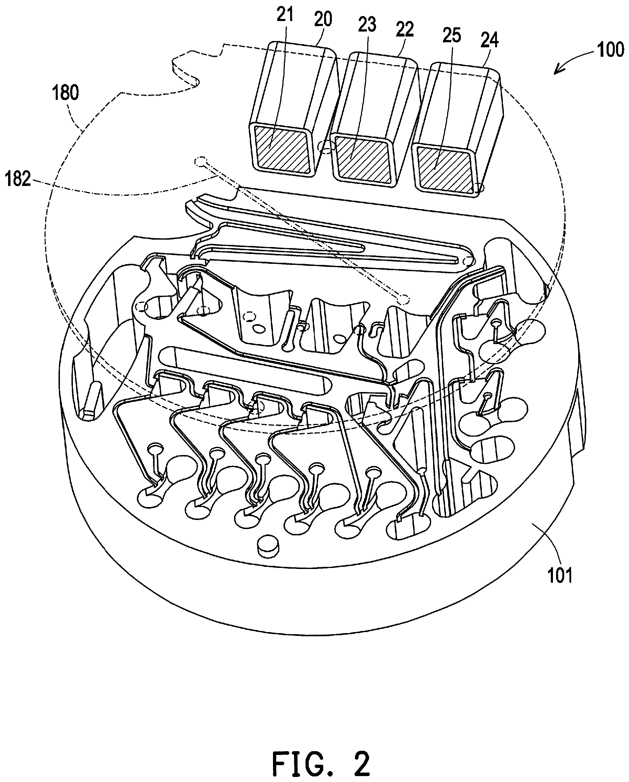

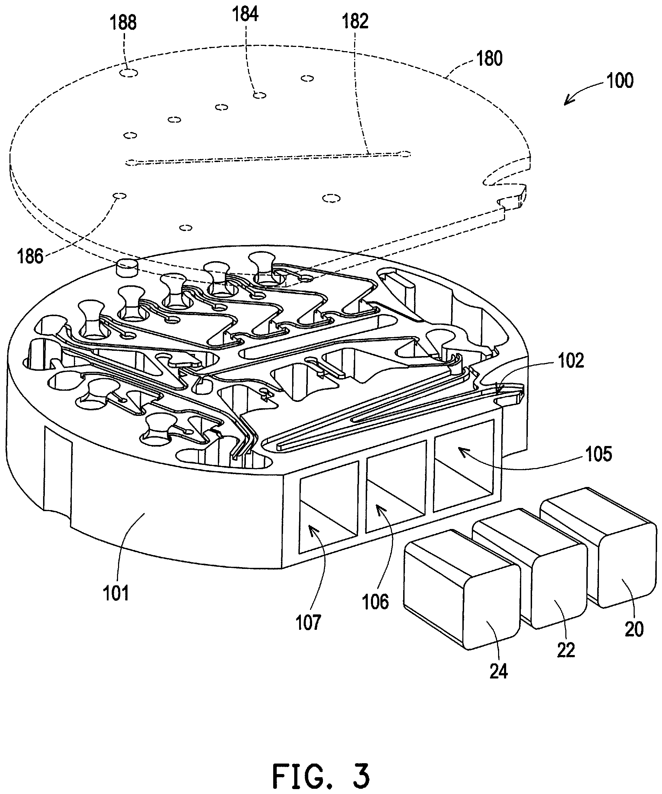

FIG. 1 is a schematic diagram of a detection cassette according to an embodiment of the invention. FIG. 2 is an exploded schematic view of the detection cassette of FIG. 1. FIG. 3 is a schematic diagram of FIG. 2 from another viewing angle.

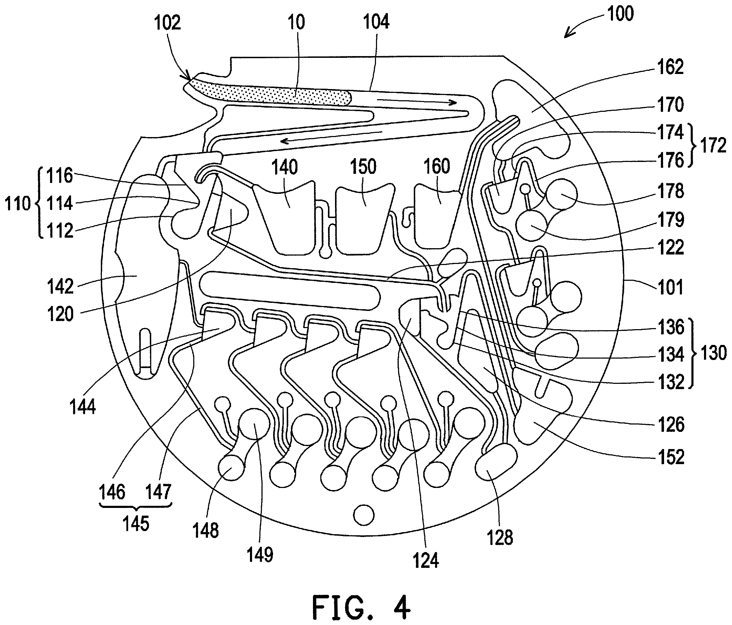

Referring to FIG. 1 to FIG. 3, the detection cassette 100 of the embodiment is adapted to test a sample 10 (FIG. 4). The detection cassette 100 includes a main body 101 and an upper cover 180. The upper cover 180 is aligned and assembled with the main body 101 through assembling holes 188. The main body 101 is provided with a plurality of tanks and flow channels. The upper cover 180 may optionally be a transparent plate, so as to clearly see the flow of the sample 10 in the main body 101.

In the embodiment, the sample 10 may flow into a place between the main body 101 and the upper cover 180 from a sample injection hole 102, and flow in the main body 101. In addition, a liquid used to mix with the sample 10 is stored within liquid cartridges 20, 22, 24 (FIG. 2).

As shown in FIG. 3, the main body 101 includes liquid cartridge insertion ports 105, 106, and 107, and the liquid cartridges 20, 22, and 24 may be respectively placed in the liquid cartridge insertion ports 105, 106, and 107. As shown in FIG. 2, the liquid cartridges 20, 22, 24 respectively include membranes 21, 23, 25. The main body 101 further includes three corresponding spikes (not shown) located deep within the liquid cartridge insertion ports 105, 106, 107.

When the liquid cartridges 20, 22 and 24 are placed in the liquid cartridge insertion ports 105, 106 and 107, the three spikes will be next to the membranes 21, 23 and 25, but are not yet contacted to the membranes 21, 23 and 25. When it is necessary to make the liquid in the liquid cartridges 20, 22, 24 to flow into the main body 101, the liquid cartridges 20, 22, 24 may be pushed inward by an external push rod (not shown) to deeper parts of the liquid cartridge insertion ports 105, 106, 107, so that the membranes 21, 23, 25 are punctured by the spikes, and the fluids stored in the liquid cartridges 20, 22, 24 flow out. As shown in FIG. 1, the fluids stored in the liquid cartridges 20, 22 and 24 respectively flow into three corresponding tanks of the main body 101 through a first through hole 141, a second through hole 151 and a third through hole 161.

The detection cassette 100 of the embodiment may be placed on a centrifugal platform (not shown) which includes, for example, a carrier turntable independently driven by a driving module and a sub turntable arranged on the carrier turntable and independently driven by another driving module. The carrier turntable is configured to provide a centrifugal force, and the sub turntable is used to adjust an angle of the detection cassette 100 relative to the carrier turntable, so that the sample 10 in the detection cassette 100 flows under the influence of the centrifugal force for the detection to be conducted.

A detailed structure and a rotation direction of the detection cassette 100 and a flow process of the sample 10 in the detection cassette 100 will be described in detail below. FIG. 4 to FIG. 14 are schematic diagrams of a flow process of the sample in the detection cassette of FIG. 1.

Referring to FIG. 4, the detection cassette 100 includes a sample injection hole 102 and a quantitative flow channel 104. The sample 10 may be injected from the sample injection hole 102, the quantitative flow channel 104 is communicated with the sample injection hole 102, and the sample 10 may be quantified in the quantitative flow channel 104. In an embodiment, the quantitative flow channel 104 may only serve as a channel through which the sample 10 flows. As shown in FIG. 4, along with the continuous injection of the sample 10, the sample 10 may move along the quantitative flow channel 104.

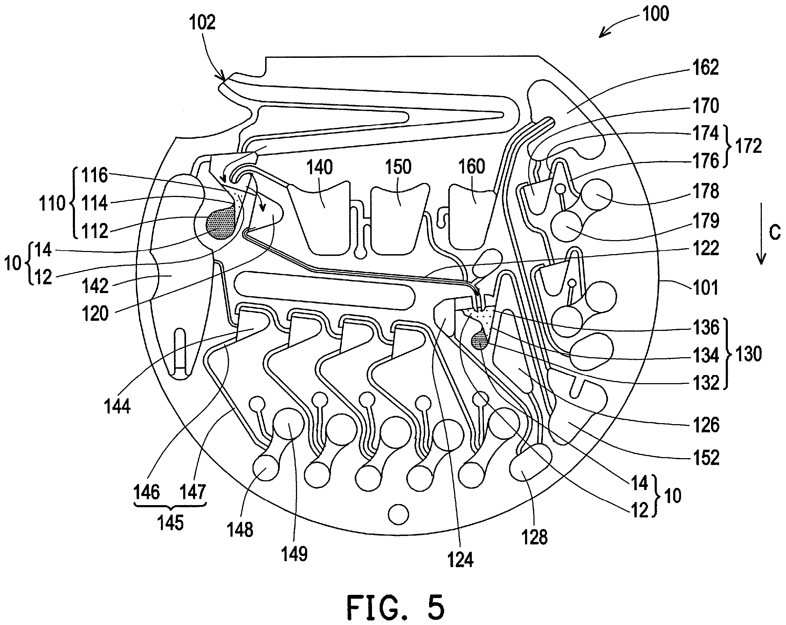

As shown in FIG. 5, the detection cassette 100 includes a first separation tank 110, a first temporary storage tank 120, a first connection flow channel 122, and a second separation tank 130. The first separation tank 110 is communicated with the quantitative flow channel 104, and the second separation tank 130 is communicated with the first separation tank 110.

To be specific, the quantitative flow channel 104 is connected between the sample injection hole 102 and the first separation tank 110. The first separation tank 110 is connected to the first temporary storage tank 120, the first temporary storage tank 120 is connected to the first connection flow channel 122, and the first connection flow channel 122 is connected to the second separation tank 130. Namely, the first temporary storage tank 120 and the first connection flow channel 122 are located between the first separation tank 110 and the second separation tank 130, and the first temporary storage tank 120 communicates between the first separation tank 110 and the first connection flow channel 122.

At such phase, a centrifugal direction C is a downward direction in FIG. 5. Therefore, as shown in FIG. 5, the sample 10 flows downward from the quantitative flow channel 104 to the first separation tank 110, a part of the sample 10 may stay in the first separation tank 110, and another part of the sample 10 may overflow from the first separation tank 110 to the second separation tank 130 through the first temporary storage tank 120 and the first connection flow channel 122.

In addition, in the embodiment, the sample 10 includes a first component 12 and a second component 14, and a density of the first component 12 is smaller than that of the second component 14. For example, the sample 10 may be whole blood, the first component 12 may be plasma, and the second component 14 may be blood cells.

Since the centrifugal direction C is a downward direction in FIG. 5, the sample 10 in the first separation tank 110 is separated into the first component 12 and the second component 14 and the sample 10 in the second separation tank 130 is separated into the first component 12 and the second component 14, under the influence of the centrifugal force.

To be specific, the first separation tank 110 includes a first bottom portion 112 and a first top portion 116. The first component 12 separated in the first separation tank 110 is adapted to be located at the first top portion 116, and the second component 14 separated in the first separation tank 110 is adapted to be located at the first bottom portion 112. A first necked section 114 may be optionally provided between the first bottom portion 112 and the first top portion 116. A width of the first necked section 114 is smaller than that of the first bottom portion 112 and the first top portion 116, so that the first bottom portion 112 and the first top portion 116 form two tanks. In other embodiments which no necked section 114 is provided between the first bottom portion 112 and the first top portion 116, the first bottom portion 112 and the first top portion 116 commonly form a single tank. The width of the first bottom portion 112 may be substantially the same as the width of the first top portion 116, or slightly smaller than the width of the first top portion 116.

Similarly, the second separation tank 130 includes a second bottom portion 132 and a second top portion 136. A second necked section 134 may be optionally provided between the second bottom portion 132 and the second top portion 136. The first component 12 separated in the second separation tank 130 is adapted to be located at the second top portion 136, and the second component 14 separated in the second separation tank 130 is adapted to be located at the second bottom portion 132. Similarly, in other embodiments, no second necked section 134 is provided between the second bottom portion 132 and the second top portion 136.

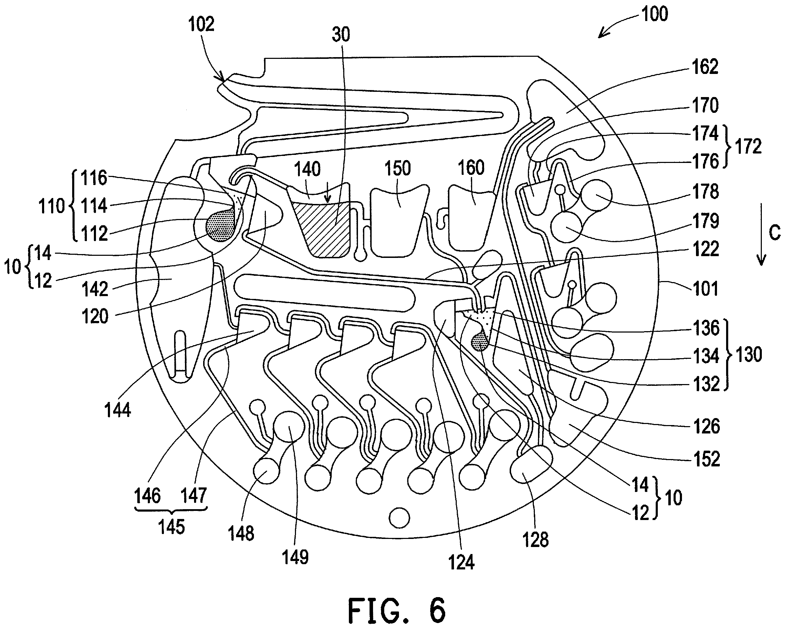

Referring to FIG. 6, in the embodiment, the detection cassette 100 further includes a first fluid tank 140. The first fluid tank 140 is adapted to contain a first fluid 30. As shown in FIG. 2 and FIG. 3, the liquid cartridge 20 (FIG. 3) located in the liquid cartridge insertion port 105 (FIG. 3) may be pushed inward by an external push rod that is not shown, and the membrane 21 (FIG. 2) of the liquid cartridge 20 is punctured by the spike in the main body 101. As shown in FIG. 6, the first fluid 30 stored in the liquid cartridge 20 is moved in the centrifugal direction C by the centrifugal force to flow into the first fluid tank 140. In the embodiment, the first fluid 30 may be a diluent, but the type of the first fluid 30 is not limited thereto.

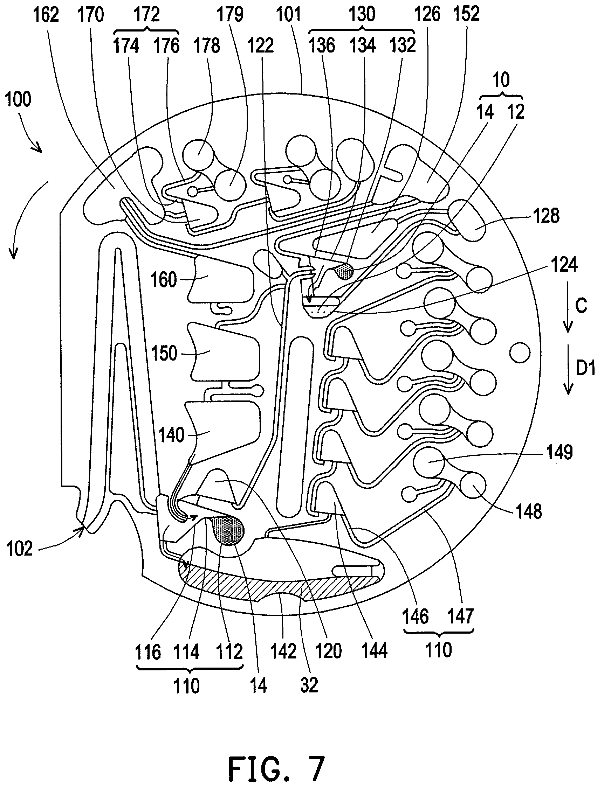

Moreover, referring to FIG. 7, the detection cassette 100 further includes a first mixing tank 142. The first mixing tank 142 is connected to the first top portion 116, and the first fluid tank 140 is connected to the first top portion 116 or the first mixing tank 142.

When the detection cassette 100 is rotated as shown in FIG. 7, the centrifugal direction C is in a first direction D1. The first mixing tank 142 is located in the first direction D1 of the first top portion 116 and the first fluid tank 140, i.e. at a lower position of FIG. 7. Namely, when the centrifugal direction C is in the first direction D1, the first mixing tank 142 is further away from a rotation center (not shown) than the first top portion 116 and the first fluid tank 140. Therefore, the first component 12 located at the first top portion 116 and the first fluid 30 located in the first fluid tank 140 flow into the first mixing tank 142, and are mixed in the first mixing tank 142 to form a first mixed solution 32.

On the other hand, the detection cassette 100 further includes a third temporary storage tank 124 communicated with the second top portion 136. The third temporary storage tank 124 is located in the first direction D1 of the second top portion 136, so that the first component 12 located at the second top portion 136 flows into the third temporary storage tank 124 and is separated from the second component 14 remained at the second bottom portion 132.

It should be noted that, generally, about 50% to 60% of the whole blood is plasma, and about 40% to 50% is blood cells. Since the second component 14 (blood cells) is to be tested after it is separated from the sample 10 in the second separation tank 130, in the embodiment, a volume of the second bottom portion 132 may be optionally smaller than a volume of the second top portion 136. Preferably, the volume of the second bottom portion 132 is less than 35% of the second separation tank 130, which may be, for example, 30%. Such design may ensure that the second bottom portion 132 contains only the second component 14. In this way, when the detection cassette 100 is rotated from the position shown in FIG. 6 to the position shown in FIG. 7, the part of the sample 10 at the second top portion 136 flows to the third temporary storage tank 124, and only the part of the sample 10 at the second bottom portion 132 is remained in the second separation tank 130, so as to prevent the first component 12 from remaining in the second separation tank 130 to interfere subsequent detection results of the second component 14.

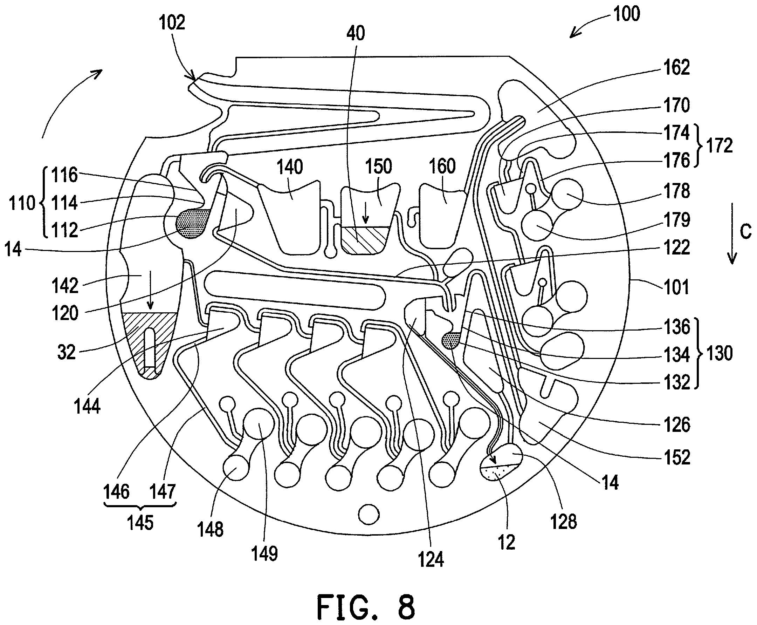

In addition, since the third temporary storage tank 124 is communicated with the overflow tank 128, when the detection cassette 100 is rotated as shown in FIG. 8, the overflow tank 128 is located in the centrifugal direction C of the third temporary storage tank 124, which is at a lower position of FIG. 8, so that the first component 12 in the third temporary storage tank 124 may flow to the overflow tank 128 under the centrifugal force. At this time, in addition to collection of the excess fluid, an optical detection may be conducted at the overflow tank 128 to detect optical background parameters of the overflowing fluid (the first component 12) before reacting with a reagent.

The detection cassette 100 further includes a second fluid tank 150. Referring to FIG. 2, FIG. 3 and FIG. 8, the liquid cartridge 22 (FIG. 3) located in the liquid cartridge insertion port 106 (FIG. 3) may be pushed inward by the external push rod, and the membrane 23 (FIG. 2) of the liquid cartridge 22 is punctured by the spike in the main body 101. As shown in FIG. 8, the second fluid 40 stored in the liquid cartridge 22 is moved in the centrifugal direction C by the centrifugal force to flow into the second fluid tank 150. The second fluid 40 may be a reagent solution for blood cells, but the type of the second fluid 40 is not limited thereto.

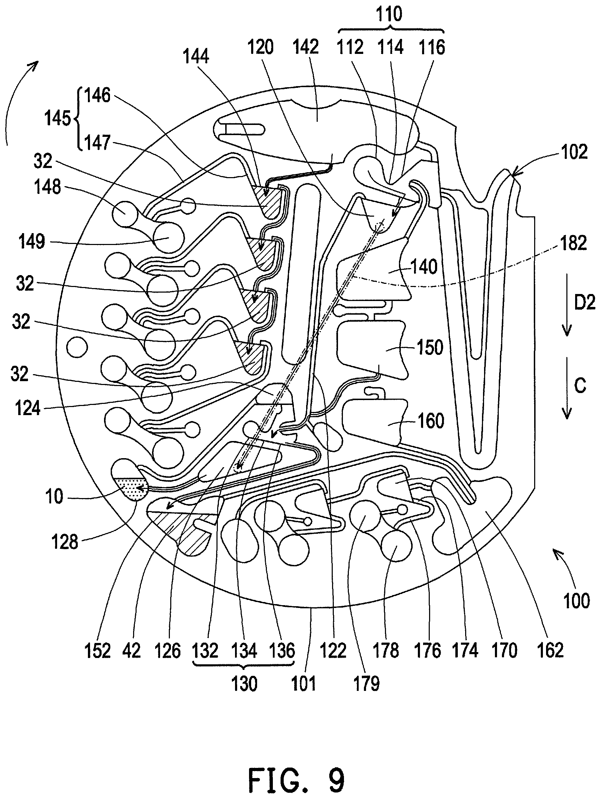

Then, referring to FIG. 9, the detection cassette 100 further includes a first quantitative tank 144, a first bending section 145 and first detection tanks 148, 149. In the embodiment, the number of the first quantitative tank 144, the first bending section 145, the first detection tank 148, and the first detection tank 149 are each plural. Certainly, the detection cassette 100 may alternatively have only a single first quantitative tank 144, a single first bending section 145, a single first detection tank 148 and a single first detection tank 149. The number of these elements is not limited thereto.

The first quantitative tank 144 is connected to the first mixing tank 142. The first bending section 145 includes a first section 146 and a second section 147 connected in a bending manner. The first section 146 is connected to the first quantitative tank 144, and the second section 147 is connected to the first detection tanks 148 and 149. As shown in FIG. 9, when the centrifugal direction C of the detection cassette 100 is in a second direction D2, the first quantitative tank 144 is in the second direction D2 of the first mixing tank 142, i.e., at a lower position of FIG. 9. The second direction D2 is the opposite direction to the first direction D1. Therefore, the first mixed solution 32 located in the first mixing tank 142 flows into the first quantitative tank 144.

In addition, in the embodiment, the first quantitative tank 144 is located in the second direction D2 of the first segment 146. Therefore, the first mixed solution 32 does not flow to the first section 146 at this phase, but flows to other first quantitative tanks 144 connected in the centrifugal direction C (lower position of FIG. 9) in sequence, so that a plurality of quantified first mixed solution 32 may be obtained for subsequent tests.

On the other hand, the detection cassette 100 further includes a second temporary storage tank 126 and a second connection flow channel 182. The second connection flow channel 182 is connected to the first temporary storage tank 120 and the second temporary storage tank 126, and the second temporary storage tank 126 is connected to the overflow tank 128. In the embodiment, the second connection flow channel 182 is located on the upper cover 180, and such design may save a space on the main body 101.

As shown in FIG. 9, the centrifugal direction C of the detection cassette 100 is in the second direction D2. The first temporary storage tank 120 is located in the second direction D2 of the first separation tank 110, and the second connection flow channel 182 and the second temporary storage tank 126 are located in the second direction D2 (a lower position of FIG. 9) of the first temporary storage tank 120. Namely, the first temporary storage tank 120 is farther from the rotation center (not shown) than the first separation tank 110, and the second connection flow channel 182 and the second temporary storage tank 126 are farther from the rotation center than the first temporary storage tank 120. Therefore, the second component 14 from the first bottom portion 112 of the first separation tank 110 may flow to the second temporary storage tank 126 along the second connection flow channel 182, and then flow to the overflow tank 128. At this time, the overflow tank 128 may be used to collect excess fluid. In this way, the first separation tank 110 and the second separation tank 130 may share the overflow tank 128, thereby saving space on the detection cassette.

Moreover, in the embodiment, the detection cassette 100 further includes a second mixing tank 152. The second fluid tank 150 is communicated with the second bottom portion 132, and the second mixing tank 152 is communicated with the second bottom portion 132. In FIG. 9, the second bottom portion 132 is located in the second direction D2 (a lower position in FIG. 9) of the second fluid tank 150, and the second mixing tank 152 is located in the second direction D2 (a lower position in FIG. 9) of the second bottom portion 132. Therefore, the second fluid 40 in the second fluid tank 150 is adapted to flow to the second bottom portion 132, so as to flow to the second mixing tank 152 together with the second component 14 located at the second bottom portion 132 to form a second mixed solution 42.

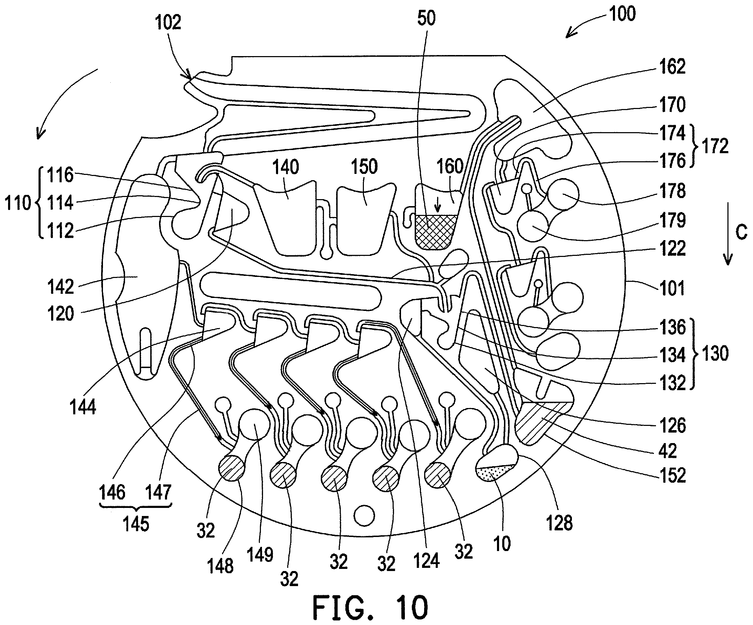

Then, referring to FIG. 10, in the embodiment, the first detection tanks 148 and 149 may be filled with a reagent or medicament, preferably a dry reagent or medicament. The detection cassette 100 is rotated so that the first bending section 145 is located in the centrifugal direction C of the first quantitative tank 144 (a lower position of FIG. 10). Therefore, the first mixed solution 32 located in the first quantitative tank 144 flows from the first bending section 145 to the first detection tank 148. The first mixed solution 32 may be mixed with the reagent or medicament in the first detection tank 148.

On the other hand, in the embodiment, the detection cassette 100 further includes a third fluid tank 160. The third fluid tank 160 is adapted to contain a third fluid 50. Referring to FIG. 2, FIG. 3 and FIG. 10, the liquid cartridge 24 (FIG. 3) located in the liquid cartridge insertion port 107 (FIG. 3) may be pushed inward by the external push rod, and the membrane 25 (FIG. 2) of the liquid cartridge 24 is punctured by the spike in the main body 101. As shown in FIG. 10, the third fluid 50 stored in the liquid cartridge 24 is moved in the centrifugal direction C by the centrifugal force and flows into the third fluid tank 160. The third fluid 50 may be another reagent liquid for blood cells, but the type of the third fluid 50 is not limited thereto.

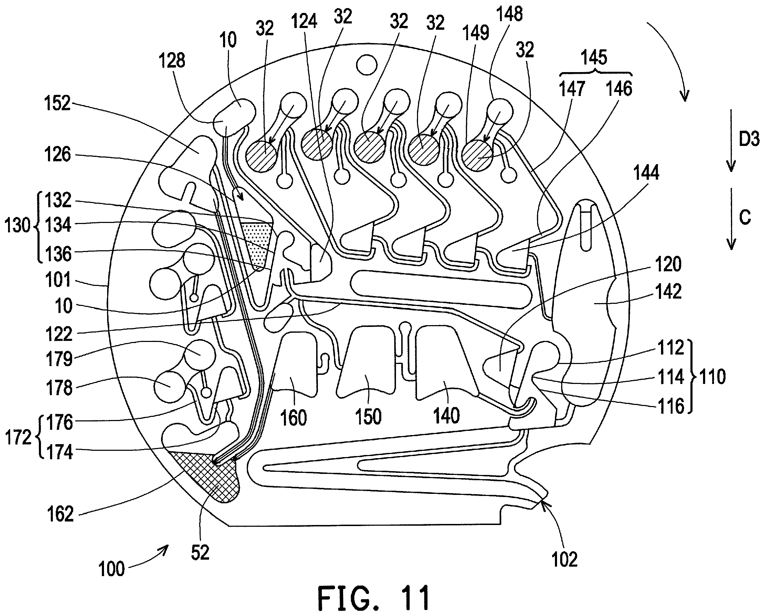

Referring to FIG. 11, the detection cassette 100 is rotated. When the centrifugal direction C of the detection cassette 100 is in a third direction D3 which is substantially orthogonal to the first direction D1 and the second direction D2, the first mixed solution 32 located in the first detection tank 148 flows to the other first detection tank 149 to fully mix the first mixed solution 32 with the reagent or medicament in the first detection tanks 148 and 149.

On the other hand, the detection cassette 100 further includes a third mixing tank 162. The third mixing tank 162 is communicated with the second mixing tank 152 and the third fluid tank 160. The third mixing tank 162 is located in the third direction D3 of the third fluid tank 160 and the second mixing tank 152. Therefore, the third fluid 50 located in the third fluid tank 160 and the second component 14 and the second fluid 40 located in the second mixing tank 152 flow to the third mixing tank 162 to form a third mixed solution 52.

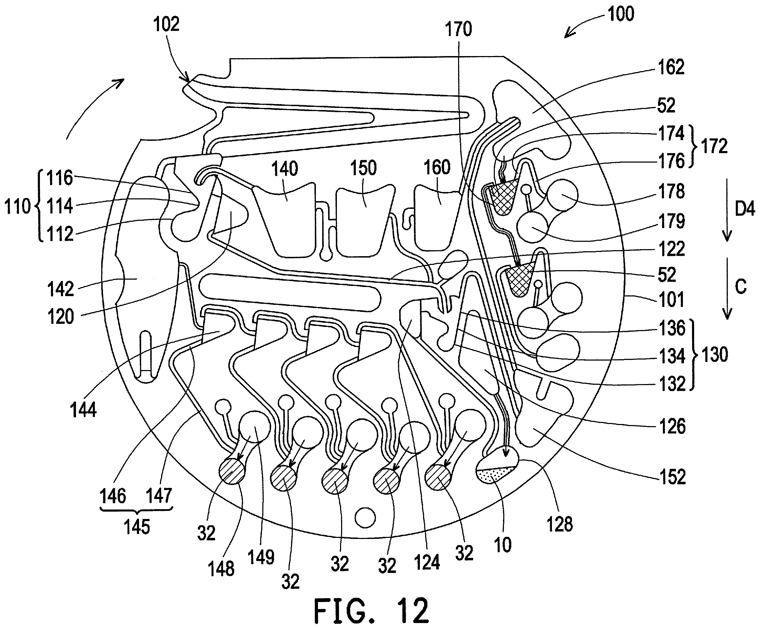

Then, referring to FIG. 12, the detection cassette 100 is rotated. When the centrifugal direction C of the detection cassette 100 is in a fourth direction D4, the first mixed solution 32 located in the first detection tank 149 flows back to the first detection tank 148, so that the first mixed solution 32 can be mixed with the reagent or medicament between the two first detection tanks 148 and 149.

On the other hand, in the embodiment, the detection cassette 100 further includes one or a plurality of second quantitative tanks 170, second bending sections 172, second detection tanks 178, and second detection tanks 179. The second quantitative tank 170 is communicated with the third mixing tank 162. The second bending section 172 includes a third section 174 and a fourth section 176 connected in a bending manner. The third section 174 is connected to the second quantitative tank 170, and the fourth section 176 is connected to the second detection tanks 178 and 179.

When the centrifugal direction C of the detection cassette 100 is in the fourth direction D4, the second quantitative tank 170 is located in the fourth direction D4 of the third mixing tank 162 and the third section 174. The fourth direction D4 is opposite to the third direction D3. Therefore, the third mixed solution 52 located in the third mixing tank 162 flows to the second quantitative tank 170.

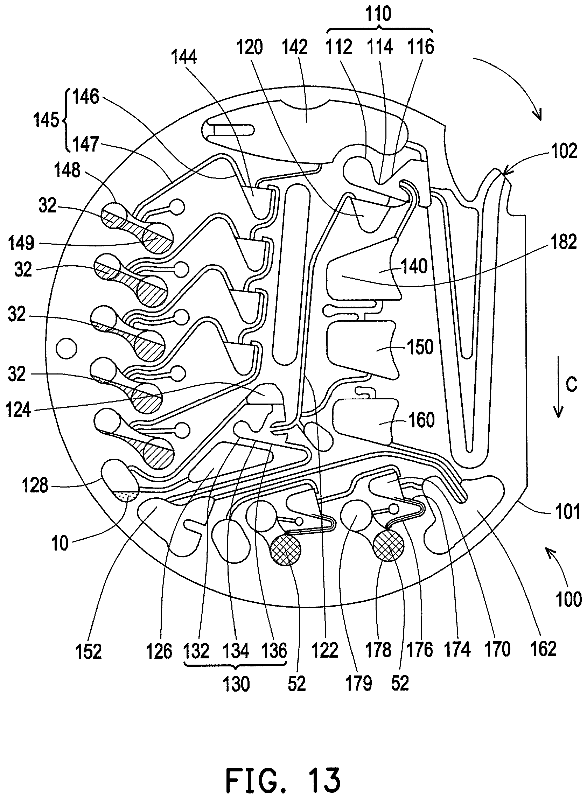

Referring to FIG. 13, the detection cassette 100 is rotated, and the first mixed solution 32 is mixed with the reagent or medicament in the first detection tanks 148 and 149. On the other hand, the third mixed solution 52 located in the second quantitative tank 170 flows from the second bending section 172 to the second detection tank 178. Similarly, the second detection tank 178 may be filled with a medicament or reagent, preferably a dry medicament or reagent, used for detecting the second component 14, but the invention is not limited thereto.

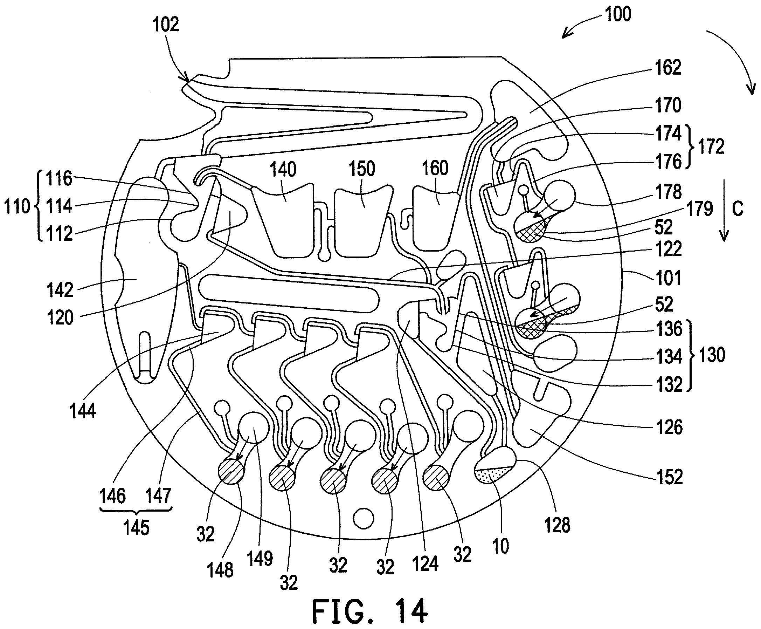

Then, referring to FIG. 14, the detection cassette 100 is rotated, and the first mixed solution 32 located in the first detection tank 149 flows to the first detection tank 148 to be mixed with the reagent or medicament of the first component 12 again. At the same time, the third mixed solution 52 located in the second detection tank 178 flows to the second detection tank 179 to be mixed with the reagent or medicament of the second component 14.

Referring back to FIG. 1, in the embodiment, the upper cover 180 further includes gas escape holes 184 and 186. The gas escape holes 184 are communicated with the first detection tanks 148 and 149, and a gas in the first detection tanks 148 and 149 (FIG. 14) may be discharged from the gas escape holes 184. The gas escape holes 186 are communicated with the second detection tanks 178 and 179, and the gas in the second detection tanks 178 and 179 (FIG. 14) may be discharged from the gas escape holes 186. As a result, the first mixed solution 32 in the first detection tanks 148 and 149 and the third mixed solution 52 in the second detection tanks 178 and 179 can flow smoothly.

The steps shown in FIG. 13 and FIG. 14 can be repeated several times to ensure that the first mixed solution 32 and the reagent or medicament are completely mixed. Once mixing of the third mixed solution 52 and the reagent or medicament is completed, detection of the first component 12 may be performed in the first detection tanks 148 and 149, and detection of the second component 14 may be performed in the second detection tanks 178 and 179. The detection is, for example, optical detection or other detections. For example, an external optical device (not shown) may be used to detect the mixtures in the first detection tanks 148, 149 and the second detection tanks 178, 179 through the transparent upper cover 180. Alternatively, the transparent upper cover 180 may be removed and then the quantified mixtures may be taken out from the detection cassette to perform other detections.

As describe above, after the sample 10 is injected into the detection cassette 100 of the embodiment, the sample 10 can be separated into the first component 12 and the second component 14 by centrifugal force by rotating the detection cassette 100 to different directions. After the first component 12 and the second component 14 are mixed with the corresponding fluids, the mixtures flow to the first detection tanks 148, 149 and the second detection tanks 178, 179. Detections of the first component 12 and the second component 14 can be respectively performed in the first detection tanks 148, 149 and the second detection tanks 178, 179. Therefore, the first component 12 and the second component 14 may be detected in one single detection cassette 100, which greatly shortens the required detection time, sample amount, and amount of the detection cassettes.

In summary, in the detection cassette of the invention, a part of the sample is adapted to enter the first separation tank from the sample injection hole of the detection cassette, and separated into the first component and the second component in the first separation tank. Another part of the sample is adapted to enter the second separation tank from the sample injection hole, and separated into the first component and the second component in the second separation tank. The first detection tank is communicated with the first separation tank, and the first component separated in the first separation tank flows to the first detection tank for detection. The second detection tank is communicated with the second separation tank, and the second component separated in the second separation tank flows to the second detection tank for detection. Therefore, the invention allows detections of the first component and the second component of one sample be conducted on one detection cassette, which effectively reduces detection cost and time, and increases utilization of the sample.

## Claims

1. A detection cassette (100), adapted to detect a sample (10), wherein the sample (10) comprises a first component (12) and a second component (14), the detection cassette (100) comprising:
a sample injection hole (102);
a first separation tank (110), communicated with the sample injection hole (102), wherein a part of the sample (10) is adapted to enter the first separation tank (110) from the sample injection hole (102), and the part of the sample (10) is separated into the first component (12) and the second component (14) in the first separation tank (110);
a second separation tank (130), communicated with the sample injection hole (102), wherein another part of the sample (10) is adapted to enter the second separation tank (130) from the sample injection hole (102), and the another part of the sample (10) is separated into the first component (12) and the second component (14) in the second separation tank (130);
a first detection tank (148, 149), communicated with the first separation tank (110), wherein the first component (12) separated in the first separation tank (110) flows to the first detection tank (148, 149) for detection; and
a second detection tank (178, 179), communicated with the second separation tank (130), **characterized in that** the second component (14) separated in the second separation tank (130) flows to the second detection tank (178, 179) for detection; and the detection cassette (100) further comprises:
a first connection flow channel (122), connected between the first separation tank (110) and the second separation tank (130).

2. The detection cassette (100) as claimed in claim 1, wherein a density of the first component (12) is less than a density of the second component (14), the first separation tank (110) comprises a first bottom portion (112) and a first top portion (116), and the first component (12) separated in the first separation tank (110) is adapted to be located at the first top portion (116), and the second component (14) separated in the first separation tank (110) is adapted to be located at the first bottom portion (112), the second separation tank (130) comprises a second bottom portion (132) and a second top portion (136), the first component (12) separated in the second separation tank (130) is adapted to be located at the second top portion (136), and the second component (14) separated in the second separation tank (130) is adapted to be located at the second bottom portion (132).

3. The detection cassette (100) as claimed in claim 2, further comprising:
a first fluid tank (140), adapted to contain a first fluid (30); and
a first mixing tank (142), connected to the first top portion (116), and the first fluid tank (140) being connected to the first top portion (116) or the first mixing tank (142), wherein
the first mixing tank (142) is located in a first direction (D1) of the first top portion (116) and the first fluid tank (140), and the first component (12) located at the first top portion (116) and the first fluid (30) located in the first fluid tank (140) are adapted to be mixed in the first mixing tank (142).

4. The detection cassette (100) as claimed in claim 3, further comprising:
a first quantitative tank (144), connected to the first mixing tank (142);
a first bending section (145), comprising a first section (146) and a second section (147) connected in a bending manner, wherein the first section (146) is connected to the first quantitative tank (144), the second section (147) is connected to the first detection tank (148, 149), wherein the first quantitative tank (144) is located in a second direction (D2) of the first mixing tank (142) and the first section (146), and the second direction (D2) is opposite to the first direction (D1).

5. The detection cassette (100) as claimed in claim 1, further comprising:
a quantitative flow channel (104), connected between the sample injection hole (102) and the first separation tank (110); and
an overflow tank (128), communicated with the second separation tank (130).

6. The detection cassette (100) as claimed in claim 5, further comprising:
a first temporary storage tank (120), communicated between the first separation tank (110) and the first connection flow channel (122), the first temporary storage tank (120) is located in a first direction (D1) of the second separation tank (130) and in a second direction (D2) of the first separation tank (110), and the second direction (D2) is opposite to the first direction (D1);
a second temporary storage tank (126), communicated with the overflow tank (128); and
a second connection flow channel (182), communicated with the first temporary storage tank (120) and the second temporary storage tank (126), wherein the second connection flow channel (182) and the second temporary storage tank (126) are located in the second direction (D2) of the first temporary storage tank (120).

7. The detection cassette (100) as claimed in claim 2, wherein there is a first necked section (114) between the first bottom portion (112) and the first top portion (116), and there is a second necked section (134) between the second bottom portion (132) and the second top portion (136).

8. The detection cassette (100) as claimed in claim 2, further comprising:
a third temporary storage tank (124), connected to the second top portion (136), wherein the third temporary storage tank (124) is located in a first direction (D1) of the second top portion (136), and the first component (12) located at the second top portion (136) is adapted to flow into the third temporary storage tank (124).

9. The detection cassette (100) as claimed in claim 3, further comprising:
a second fluid tank (150), adapted to contain a second fluid (40), and communicated with the second bottom portion (132); and
a second mixing tank (152), communicated with the second bottom portion (132), wherein
the second bottom portion (132) is located in a second direction (D2) of the second fluid tank (150), the second direction (D2) is opposite to the first direction (D1), the second mixing tank (152) is located in the second direction (D2) of the second bottom portion (132), and the second fluid (40) in the second fluid tank (150) is adapted to flow to the second bottom portion (132), so as to flow to the second mixing tank (152) together with the second component (14) located at the second bottom portion (132).

10. The detection cassette (100) as claimed in claim 9, further comprising:
a third fluid tank (160), adapted to contain a third fluid (50); and
a third mixing tank (162), communicated with the second mixing tank (152) and the third fluid tank (160), wherein the third mixing tank (162) is located in a third direction (D3) of the third fluid tank (160) and the second mixing tank (152), the third direction (D3) is orthogonal to the first direction (D1) and the second direction (D2), and the third fluid (50) in the third fluid tank (160) and the second component (14) and the second fluid (40) in the second mixing tank (152) are adapted to flow to the third mixing tank (162).

11. The detection cassette (100) as claimed in claim 10, further comprising:
a second quantitative tank (170), communicated with the third mixing tank (162); and
a second bending section (172), comprising a third section (174) and a fourth section (176) connected in a bending manner, wherein the third section (174) is connected to the second quantitative tank (170), the fourth section (176) is connected to the second detection tank (178, 179), the second quantitative tank (170) is located in a fourth direction (D4) of the third mixing tank (162) and the third section (174), and the fourth direction (D4) is opposite to the third direction (D3).

12. A detection system, comprising:
a centrifugal platform having a carrier turntable driven by a driving module; and
a detection cassette (100) as claimed in one of claims 1-11 placed on the carrier turntable.

13. The detection system as claimed in claim 12, wherein the centrifugal platform further comprises a sub turntable arranged on the carrier turntable and driven by another driving module,
wherein the detection cassette (100) is placed on the sub turntable.

## Patentansprüche

1. Detektionskassette (100), die angepasst ist, um eine Probe (10) zu detektieren, wobei die Probe (10) einen ersten Bestandteil (12) und einen zweiten Bestandteil (14) umfasst, wobei die Detektionskassette (100) umfasst:
eine Probeninjektionsöffnung (102);
einen ersten Trennungsbehälter (110), der mit der Probeninjektionsöffnung (102) in Verbindung ist, wobei ein Teil der Probe (10) angepasst ist, um von der Probeninjektionsöffnung (102) in den ersten Trennungsbehälter (110) einzutreten, und der Teil der Probe (10) in dem ersten Trennungsbehälter (110) in den ersten Bestandteil (12) und den zweiten Bestandteil (14) getrennt wird;
einen zweiten Trennungsbehälter (130), der mit der Probeninjektionsöffnung (102) in Verbindung ist, wobei ein anderer Teil der Probe (10) angepasst ist, um von der Probeninjektionsöffnung (102) in den zweiten Trennungsbehälter (130) einzutreten, und der andere Teil der Probe (10) in dem zweiten Trennungsbehälter (130) in den ersten Bestandteil (12) und den zweiten Bestandteil (14) getrennt wird;
einen ersten Detektionsbehälter (148, 149), der mit dem ersten Trennungsbehälter (110) in Verbindung ist, wobei der erste Bestandteil (12), der in dem ersten Trennungsbehälter (110) getrennt wird, zu dem ersten Detektionsbehälter (148, 149) zur Detektion fließt; und
einen zweiten Detektionsbehälter (178, 179), der mit dem zweiten Trennungsbehälter (130) in Verbindung ist, **dadurch gekennzeichnet, dass** der zweite Bestandteil (14), der in dem zweiten Trennungsbehälter (130) abgetrennt wird, zu dem zweiten Detektionsbehälter (178, 179) zur Detektion fließt; und die Detektionskassette (100) ferner umfasst:
einen ersten Verbindungsströmungskanal (122), der zwischen dem ersten Trennungsbehälter (110) und dem zweiten Trennungsbehälter (130) verbunden ist.

2. Detektionskassette (100) gemäß Anspruch 1, wobei eine Dichte des ersten Bestandteils (12) geringer ist als eine Dichte des zweiten Bestandteils (14), der erste Trennungsbehälter (110) einen ersten unteren Abschnitt (112) und einen ersten oberen Abschnitt (116) umfasst, und der erste Bestandteil (12), der in dem ersten Trennungsbehälter (110) getrennt wird, so angepasst ist, dass er sich an dem ersten oberen Abschnitt (116) befindet, und der zweite Bestandteil (14), der in dem ersten Trennungsbehälter (110) getrennt wird, so angepasst ist, dass er sich an dem ersten unteren Abschnitt (112) befindet, der zweite Trennungsbehälter (130) einen zweiten unteren Abschnitt (132) und einen zweiten oberen Abschnitt (136) umfasst, der erste Bestandteil (12), der in dem zweiten Trennungsbehälter (130) getrennt wird, so angepasst ist, dass er sich an dem zweiten oberen Abschnitt (136) befindet, und der zweite Bestandteil (14), der in dem zweiten Trennungsbehälter (130) getrennt wird, so angepasst ist, dass er sich an dem zweiten unteren Abschnitt (132) befindet.

3. Detektionskassette (100) gemäß Anspruch 2, ferner umfassend:
einen ersten Fluidbehälter (140), der angepasst ist, um ein erstes Fluid (30) zu enthalten; und
einen ersten Mischbehälter (142), der mit dem ersten oberen Abschnitt (116) verbunden ist, und wobei der erste Fluidbehälter (140) mit dem ersten oberen Abschnitt (116) oder dem ersten Mischbehälter (142) verbunden ist, wobei
der erste Mischbehälter (142) sich in einer ersten Richtung (D1) des ersten oberen Abschnitts (116) und des ersten Fluidbehälters (140) befindet, und der erste Bestandteil (12), der sich an dem ersten oberen Abschnitt (116) befindet, und das erste Fluid (30), das sich in dem ersten Fluidbehälter (140) befindet, angepasst sind, in dem ersten Mischbehälter (142) gemischt zu werden.

4. Detektionskassette (100) gemäß Anspruch 3, ferner umfassend:
einen ersten Mengenbehälter (144), der mit dem ersten Mischbehälter (142) verbunden ist;
einen ersten Biegeabschnitt (145), der einen ersten Abschnitt (146) und einen zweiten Abschnitt (147) umfasst, die auf gebogene Weise verbunden sind, wobei der erste Abschnitt (146) mit dem ersten Mengenbehälter (144) verbunden ist, der zweite Abschnitt (147) mit dem ersten Detektionsbehälter (148, 149) verbunden ist, wobei der erste Mengenbehälter (144) sich in einer zweiten Richtung (D2) des ersten Mischbehälters (142) und des ersten Abschnitts (146) befindet, und die zweite Richtung (D2) der ersten Richtung (D1) entgegengesetzt ist.

5. Detektionskassette (100) gemäß Anspruch 1, ferner umfassend:
einen Mengenströmungskanal (104), der zwischen der Probeneinspritzöffnung (102) und dem ersten Trennungsbehälter (110) verbunden ist; und
einen Überlaufbehälter (128), der mit dem zweiten Trennungsbehälter (130) in Verbindung ist.

6. Detektionskassette (100) gemäß Anspruch 5, die ferner umfasst
einen ersten Zwischenspeicherbehälter (120), der zwischen dem ersten Trennungsbehälter (110) und dem ersten Verbindungsströmungskanal (122) in Verbindung ist, wobei sich der erste Zwischenspeicherbehälter (120) in einer ersten Richtung (D1) des zweiten Trennungsbehälters (130) und in einer zweiten Richtung (D2) des ersten Trennungsbehälters (110) befindet und die zweite Richtung (D2) entgegengesetzt zur ersten Richtung (D1) ist;
einen zweiten Zwischenspeicherbehälter (126), der mit dem Überlaufbehälter (128) in Verbindung ist; und
einen zweiten Verbindungsströmungskanal (182), der mit dem ersten Zwischenspeicherbehälter (120) und dem zweiten Zwischenspeicherbehälter (126) in Verbindung ist, wobei sich der zweite Verbindungsströmungskanal (182) und der zweite Zwischenspeicherbehälter (126) in der zweiten Richtung (D2) des ersten Zwischenspeicherbehälters (120) befinden.

7. Detektionskassette (100) gemäß Anspruch 2, wobei zwischen dem ersten unteren Abschnitt (112) und dem ersten oberen Abschnitt (116) ein erster verengter Abschnitt (114) und zwischen dem zweiten unteren Abschnitt (132) und dem zweiten oberen Abschnitt (136) ein zweiter verengter Abschnitt (134) vorhanden ist.

8. Detektionskassette (100) gemäß Anspruch 2, ferner umfassend:
einen dritten Zwischenspeicherbehälter (124), der mit dem zweiten oberen Abschnitt (136) verbunden ist, wobei sich der dritte Zwischenspeicherbehälter (124) in einer ersten Richtung (D1) des zweiten oberen Abschnitts (136) befindet, und der erste Bestandteil (12), der sich an dem zweiten oberen Abschnitt (136) befindet, angepasst ist, um in den dritten Zwischenspeicherbehälter (124) zu fließen.

9. Detektionskassette (100) gemäß Anspruch 3, ferner umfassend:
einen zweiten Fluidbehälter (150), der angepasst ist, um ein zweites Fluid (40) zu enthalten und mit dem zweiten unteren Abschnitt (132) in Verbindung ist; und
einen zweiten Mischbehälter (152), der mit dem zweiten unteren Abschnitt (132) in Verbindung ist, wobei
sich der zweite untere Abschnitt (132) in einer zweiten Richtung (D2) des zweiten Fluidbehälters (150) befindet, wobei die zweite Richtung (D2) der ersten Richtung (D1) entgegengesetzt ist, der zweite Mischbehälter (152) sich in der zweiten Richtung (D2) des zweiten unteren Abschnitts (132) befindet, und das zweite Fluid (40) in dem zweiten Fluidbehälter (150) angepasst ist, um zu dem zweiten unteren Abschnitt (132) zu fließen, so dass es zusammen mit dem zweiten Bestandteil (14), der sich an dem zweiten unteren Abschnitt (132) befindet, zu dem zweiten Mischbehälter (152) fließt.

10. Detektionskassette (100) gemäß Anspruch 9, ferner umfassend:
einen dritten Fluidbehälter (160), der angepasst ist, um ein drittes Fluid (50) zu enthalten; und
einen dritten Mischbehälter (162), der mit dem zweiten Mischbehälter (152) und dem dritten Fluidbehälter (160) in Verbindung ist, wobei sich der dritte Mischbehälter (162) in einer dritten Richtung (D3) des dritten Fluidbehälters (160) und des zweiten Mischbehälters (152) befindet, die dritte Richtung (D3) orthogonal zu der ersten Richtung (D1) und der zweiten Richtung (D2) ist, und das dritte Fluid (50) in dem dritten Fluidbehälter (160) und der zweite Bestandteil (14) und das zweite Fluid (40) in dem zweiten Mischbehälter (152) angepasst sind, um zu dem dritten Mischbehälter (162) zu fließen.

11. Detektionskassette (100) gemäß Anspruch 10, die ferner umfasst:
einen zweiten Mengenbehälter (170), der mit dem dritten Mischbehälter (162) in Verbindung ist; und
einen zweiten Biegeabschnitt (172), der einen dritten Abschnitt (174) und einen vierten Abschnitt (176) umfasst, die auf gebogene Weise verbunden sind, wobei der dritte Abschnitt (174) mit dem zweiten Mengenbehälter (170) verbunden ist, der vierte Abschnitt (176) mit dem zweiten Detektionsbehälter (178, 179) verbunden ist, der zweite Mengenbehälter (170) sich in einer vierten Richtung (D4) des dritten Mischbehälters (162) und des dritten Abschnitts (174) befindet und die vierte Richtung (D4) entgegengesetzt zur dritten Richtung (D3) ist.

12. Detektionssystem, umfassend:
eine Zentrifugalplattform, die einen Trägerteller aufweist, der durch ein Antriebsmodul angetrieben wird; und
eine Detektionskassette (100) gemäß einem der Ansprüche 1 bis 11, die auf dem Trägerdrehteller angeordnet ist.

13. Detektionssystem gemäß Anspruch 12, wobei die Zentrifugalplattform ferner einen Unterdrehteller umfasst, der auf dem Trägerdrehteller angebracht ist und von einem anderen Antriebsmodul angetrieben wird, wobei die Detektionskassette (100) auf dem Unterdrehteller angeordnet ist.

## Revendications

1. Cassette de détection (100) adaptée pour détecter un échantillon (10), dans laquelle l'échantillon (10) comprend un premier composant (12) et un deuxième composant (14), la cassette de détection (100) comprenant :
un trou d'injection d'échantillon (102) ;
un premier réservoir de séparation (110) en communication avec le trou d'injection d'échantillon (102), dans laquelle une partie de l'échantillon (10) est adaptée pour entrer dans le premier réservoir de séparation (110) par le trou d'injection d'échantillon (102), et la partie de l'échantillon (10) est séparée en le premier composant (12) et le deuxième composant (14) dans le premier réservoir de séparation (110) ;
une deuxième réservoir de séparation (130) en communication avec le trou d'injection d'échantillon (102), dans laquelle une autre partie de l'échantillon (10) est adaptée pour entrer dans le deuxième réservoir de séparation (130) à partir du trou d'injection d'échantillon (102), et l'autre partie de l'échantillon (10) est séparée dans le premier composant (12) et le deuxième composant (14) dans le deuxième réservoir de séparation (130) ;
un premier réservoir de détection (148, 149) en communication avec le premier réservoir de séparation (110), dans laquelle le premier composant (12) séparée dans le premier réservoir de séparation (110) s'écoule vers le premier réservoir de détection (148, 149) pour la détection ; et
un deuxième réservoir de détection (178, 179) en communication avec le deuxième réservoir de séparation (130), **caractérisée en ce que** le deuxième composant (14) séparé dans le deuxième réservoir de séparation (130) s'écoule vers le deuxième réservoir de détection (178, 179) pour la détection ; et la cassette de détection (100) comprend en outre :
un premier canal d'écoulement de raccordement (122), raccordé entre le premier réservoir de séparation (110) et le deuxième réservoir de séparation (130).

2. Cassette de détection (100) selon la revendication 1, dans laquelle une densité du premier composant (12) est inférieure à une densité du deuxième composant (14), le premier réservoir de séparation (110) comprend une première portion basse (112) et une première portion haute (116), et le premier composant (12) séparé dans le premier réservoir de séparation (110) est adapté pour se trouver au niveau de la première portion haute (116), et le deuxième composant (14) séparé dans le premier réservoir de séparation (110) est adapté pour se trouver au niveau de la première portion basse (112), le deuxième réservoir de séparation (130) comprend une deuxième portion basse (132) et une deuxième portion haute (136), le premier composant (12) séparé dans le deuxième réservoir de séparation (130) est adapté pour se trouver au niveau de la deuxième portion haute (136), et le deuxième composant (14) séparé dans le deuxième réservoir de séparation (130) est adapté pour se trouver au niveau de la deuxième portion basse (132).

3. Cassette de détection (100) selon la revendication 2, comprenant en outre :
un premier réservoir de fluide (140) adapté pour contenir un premier fluide (30) ; et
un premier réservoir de mélange (142) relié à première portion haute (116), et le premier réservoir de fluide (140) étant relié à la première portion haute (116) ou au premier réservoir de mélange (142), dans laquelle
le premier réservoir de mélange (142) se trouve dans une première direction (D1) de la première portion haute (116) et du premier réservoir de fluide (140), et le premier composant (12) situé au niveau de la première portion haute (116) et le premier fluide (30) situé dans le premier réservoir de fluide (140) sont adaptés pour être mélangés dans le premier réservoir de mélange (142).

4. Cassette de détection (100) selon la revendication 3, comprenant en outre :
un premier réservoir quantitatif (144) relié au premier réservoir de mélange (142) ;
une première section courbée (145) comprenant une première section (146) et une deuxième section (147) reliées de façon courbée, dans laquelle la première section (146) est reliée au premier réservoir quantitatif (144), la deuxième section (147) est reliée au premier réservoir de détection (148, 149), dans laquelle le premier réservoir quantitatif (144) se trouve dans une deuxième direction (D2) du premier réservoir de mélange (142) et de la première section (146), et la deuxième direction (D2) est opposée à la première direction (D1).

5. Cassette de détection (100) selon la revendication 1, comprenant en outre :
un canal d'écoulement quantitatif (104) relié entre le trou d'injection d'échantillon (102) et le premier réservoir de séparation (110) ; et
un réservoir de trop-plein (128) en communication avec le deuxième réservoir de séparation (130).

6. Cassette de détection (100) selon la revendication 5, comprenant en outre :
un premier réservoir de stockage temporaire (120) en communication entre le premier réservoir de séparation (110) et le premier canal d'écoulement de raccordement (122), le premier réservoir de stockage temporaire (120) se trouve dans une première direction (D1) du deuxième réservoir de séparation (130) et dans une deuxième direction (D2) du premier réservoir de séparation (110), et la deuxième direction (D2) est opposée à la première direction (D1) ;
un deuxième réservoir de stockage temporaire (126) en communication avec le réservoir de trop-plein (128) ; et
un deuxième canal d'écoulement de raccordement (182) en communication avec le premier réservoir de stockage temporaire (120) et le deuxième réservoir de stockage temporaire (126), dans laquelle le deuxième canal d'écoulement de raccordement (182) et le deuxième réservoir de stockage temporaire (126) se trouvent dans la deuxième direction (D2) du premier réservoir de stockage temporaire (120).

7. Cassette de détection (100) selon la revendication 2, dans laquelle il est prévu une première section à col (114) entre la première portion basse (112) et la première portion haute (116), et il est prévu une deuxième section à col (134) entre la deuxième portion basse (132) et la deuxième portion haute (136).

8. Cassette de détection (100) selon la revendication 2, comprenant en outre :
un troisième réservoir de stockage temporaire (124) relié à la deuxième portion haute (136), dans laquelle le troisième réservoir de stockage temporaire (124) se trouve dans une première direction (D1) de la deuxième portion haute (136), et le premier composant (12) situé au niveau de la deuxième portion haute (136) est adapté pour s'écouler dans le troisième réservoir de stockage temporaire (124).

9. Cassette de détection (100) selon la revendication 3, comprenant en outre :
un deuxième réservoir de fluide (150) adapté pour contenir un deuxième fluide (40), et en communication avec la deuxième portion basse (132) ; et
un deuxième réservoir de mélange (152) en communication avec la deuxième portion basse (132), dans laquelle
la deuxième portion basse (132) se trouve dans une deuxième direction (D2) du deuxième réservoir de fluide (150), la deuxième direction (D2) est opposée à la première direction (D1), le deuxième réservoir de mélange (152) se trouve dans la deuxième direction (D2) de la deuxième portion basse (132), et le deuxième fluide (40) dans le deuxième réservoir de fluide (150) est adapté pour s'écouler vers la deuxième portion basse (132), de manière à s'écouler vers le deuxième réservoir de mélange (152) ensemble avec le deuxième composant (14) situé au niveau de la deuxième portion basse (132).

10. Cassette de détection (100) selon la revendication 9, comprenant en outre :
un troisième réservoir de fluide (160) adapté pour contenir un troisième fluide (50) ; et
un troisième réservoir de mélange (162) en communication avec le deuxième réservoir de mélange (152) et le troisième réservoir de fluide (160), dans laquelle le troisième réservoir de mélange (162) se trouve dans une troisième direction (D3) du troisième réservoir de fluide (160) et du deuxième réservoir de mélange (152), la troisième direction (D3) est orthogonale à la première direction (D1) et à la deuxième direction (D2), et le troisième fluide (50) dans le troisième réservoir de fluide (160) et le deuxième composant (14) et deuxième fluide (40) dans le deuxième réservoir de mélange (152) sont adaptés pour s'écouler vers le troisième réservoir de mélange (162).

11. Cassette de détection (100) selon la revendication 10, comprenant en outre :
un deuxième réservoir quantitatif (170) en communication avec le troisième réservoir de mélange (162) ; et
une deuxième section courbée (172) comprenant une troisième section (174) et une quatrième section (176) reliées d'une manière courbée, dans laquelle la troisième section (174) est reliée au deuxième réservoir quantitatif (170), la quatrième section (176) est reliée au deuxième réservoir de détection (178, 179), le deuxième réservoir quantitatif (170) se trouve dans une quatrième direction (D4) du troisième réservoir de mélange (162) et de la troisième section (174), et la quatrième direction (D4) est opposée à la troisième direction (D3).

12. Système de détection, comprenant :
une plateforme centrifuge comportant un plateau tournant de support entraîné par un module d'entraînement ; et
une cassette de détection (100) selon l'une des revendications 1 à 11, placée sur le plateau tournant de support.

13. Système de détection selon la revendication 12, dans lequel la plateforme centrifuge comprend en outre un plateau tournant secondaire disposé sur le plateau tournant de support et entraîné par un autre module d'entraînement,
dans lequel la cassette de détection (100) est placée sur le plateau tournant secondaire.
